(19) Europäisches Patentamt
European Patent Office
Office européen des brevets

(11) **EP 4 311 519 A1**

(12) **EUROPÄISCHE PATENTANMELDUNG**

(43) Veröffentlichungstag:
**31.01.2024  Patentblatt 2024/05**

(21) Anmeldenummer: **22187237.7**

(22) Anmeldetag: **27.07.2022**

(51) Internationale Patentklassifikation (IPC):
*A61C 13/08* (2006.01)   *A61B 5/103* (2006.01)
*G01J 3/28* (2006.01)   *G01J 3/50* (2006.01)

(52) Gemeinsame Patentklassifikation (CPC):
**G01J 3/508; A61B 5/0088; A61B 5/1032;**
**A61B 5/4547; G01J 3/28; G01J 3/501;** A61C 9/006

(84) Benannte Vertragsstaaten:
**AL AT BE BG CH CY CZ DE DK EE ES FI FR GB**
**GR HR HU IE IS IT LI LT LU LV MC MK MT NL NO**
**PL PT RO RS SE SI SK SM TR**
Benannte Erstreckungsstaaten:
**BA ME**
Benannte Validierungsstaaten:
**KH MA MD TN**

(71) Anmelder: **Ivoclar Vivadent AG**
**9494 Schaan (LI)**

(72) Erfinder:
• **Niedrig, Christian**
  **9478 Azmoos (CH)**
• **Reinhardt, Jonas**
  **7206 Igis (CH)**

(74) Vertreter: **Baldus, Oliver**
**Splanemann**
**Rumfordstrasse 7**
**80469 München (DE)**

(54) **FARBMESSUNG MIT STREIFENLICHT**

(57)     Die vorliegende Erfindung betrifft ein Verfahren zur ortsaufgelösten Farbbestimmung, mit den Schritten eines Projizierens (S101) eines ersten Streifenlichtmusters mit einer ersten Lichtwellenlänge auf ein Dentalobjekt; eines Erfassens (S102) eines ersten ortsaufgelösten optischen Parametersatzes auf Basis des reflektierten oder remittierten ersten Streifenlichtmusters; eines Projizierens (S103) eines zweiten Streifenlichtmusters mit einer zweiten Lichtwellenlänge auf das Dentalobjekt; eines Erfassens (S104) eines zweiten ortsaufgelösten optischen Parametersatzes auf Basis des reflektierten oder remittierten zweiten Streifenlichtmusters; und eines Berechnens (S105) eines dritten ortsaufgelösten optischen Parametersatzes bei einer dritten Lichtwellenlänge auf Basis des ersten und des zweiten ortsaufgelösten optischen Parametersatzes.

Fig. 1

**Beschreibung**

Die vorliegende Erfindung betrifft ein Verfahren zur ortsaufgelösten Farbbestimmung und ein Farbbestimmungssystem zur ortsaufgelösten Farbbestimmung.

Falls eine Farbmessung mit einem Intraoralscanner an einem Zahn realisiert werden soll, besteht einerseits das Problem, dass der Bauraum innerhalb des Intraoralscanners begrenzt ist und nur eine geringe Anzahl von Lichtquellen in einem Gehäuse angeordnet werden kann, beispielsweise für die Farben Rot, Grün und Blau. Auch wenn zukünftige Lichtquellen leistungsstärker und kleiner werden, ändert sich nichts daran. Je kleiner die Anzahl der Lichtquellen ist, desto geringer ist der erforderliche Bauraum innerhalb des Intraoralscanners. Für eine möglichst genaue Farbbestimmung werden andererseits jedoch möglichst zahlreiche Lichtquellen mit jeweils unterschiedlicher Lichtwellenlänge benötigt, um bei vielen Wellenlängen einen Farbmesswert zu erhalten.

Ein Spektroradiometer ist beispielsweise ein Spektrometer, das unterschiedliche Wellenlängen und Amplituden von emittiertem Licht misst. Diese Messung ist jedoch nicht ortsaufgelöst über eine Probenfläche möglich, sondern nur für das insgesamt in das Gerät eintretende Licht.

Es ist daher die technische Aufgabe der vorliegenden Erfindung, eine ortsaufgelöste und präzise Farbestimmung mit möglichst wenigen Lichtquellen durchzuführen.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zur ortsaufgelösten Farbbestimmung gelöst, mit den Schritten eines Projizierens eines ersten Streifenlichtmusters mit einer ersten Lichtwellenlänge auf ein Dentalobjekt; eines Erfassens eines ersten ortsaufgelösten optischen Parametersatzes auf Basis des reflektierten oder remittierten ersten Streifenlichtmusters; eines Projizierens eines zweiten Streifenlichtmusters mit einer zweiten Lichtwellenlänge auf das Dentalobjekt; eines Erfassens eines zweiten ortsaufgelösten optischen Parametersatzes auf Basis des reflektierten oder remittierten zweiten Streifenlichtmusters; und eines Berechnens eines dritten ortsaufgelösten optischen Parametersatzes bei einer dritten Lichtwellenlänge auf Basis des ersten und des zweiten ortsaufgelösten optischen Parametersatzes. Das Verfahren kann auch auf Grundlage weiterer Streifenlichtmuster mit weiteren Lichtwellenlängen durchgeführt werden, so dass sich seine Genauigkeit bei der Bestimmung des dritten ortsaufgelösten optischen Parametersatzes weiter verbessert. Der Parametersatz kann einen oder mehrere Parameter umfassen, die die optischen Eigenschaften des Dentalobjekts an unterschiedlichen Orten der Oberfläche beschreiben.

Durch das Verfahren wird der technische Vorteil erreicht, dass weitere ortsaufgelöste optische Parametersätze bei jeder beliebigen Lichtwellenlänge erhalten werden können. Auf diese Weise kann für jeden Ort auf der Oberfläche des Dentalobjektes ein vollständiges Farbspektrum berechnet werden. Durch die Berechnung von ortsaufgelösten optischen Parametersätzen lässt sich die Anzahl der Lichtquellen verringern, da auf eine Messung bei dieser Lichtwellenlänge verzichtet werden kann. Trotzdem können ortsaufgelöste Farbwerte mit hoher Genauigkeit ermittelt werden.

In einer technisch vorteilhaften Ausführungsform des Verfahrens wird der dritte ortsaufgelöste optische Parametersatz aus dem ersten und dem zweiten ortsaufgelösten optischen Parametersatz extrapoliert. Dadurch wird beispielsweise der technische Vorteil erreicht, dass der dritte ortsaufgelöste optische Parametersatz auf einfache Weise und mit geringem Rechenaufwand ermittelt werden kann. Dies ist besonders dann von Vorteil, wenn der dritte ortsaufgelöste optische Parametersatz in geringem Wellenlängenabstand außerhalb des Intervalls der ersten und der zweiten Lichtwellenlänge berechnet werden soll.

In einer weiteren technisch vorteilhaften Ausführungsform des Verfahrens wird der dritte ortsaufgelöste optische Parametersatz zwischen dem ersten und dem zweiten ortsaufgelösten optischen Parametersatz interpoliert. Dadurch wird beispielsweise der technische Vorteil erreicht, dass der dritte ortsaufgelöste optische Parametersatz auf einfache Weise und mit geringem Rechenaufwand ermittelt werden kann. Dies ist besonders dann von Vorteil, wenn das Intervall zwischen der ersten und der zweiten Lichtwellenlänge klein ist.

In einer weiteren technisch vorteilhaften Ausführungsform des Verfahrens wird der dritte Parametersatz durch ein Fitverfahren auf Basis des ersten und des zweiten ortsaufgelösten optischen Parametersatzes bestimmt. Durch das Fitverfahren wird ein allgemeiner funktionaler Zusammenhang eines optischen Parameters in Abhängigkeit der Lichtwellenlänge, der für das Dentalobjekt zu erwarten ist, auf Basis der gemessenen optischen Parameter bei der ersten und der zweiten Lichtwellenlänge angepasst. Dadurch wird beispielsweise der technische Vorteil erreicht, dass sich die Farbwerte mit einer hohen Genauigkeit bestimmen lassen.

In einer weiteren technisch vorteilhaften Ausführungsform des Verfahrens wird ein vorgegebener spektraler Verlauf an den ersten und/oder den zweiten ortsaufgelösten optischen Parametersatz angepasst, um den dritten ortsaufgelösten optischen Parametersatz zu erhalten. Beispielsweise kann der dritte ortsaufgelöste optische Parametersatz dadurch erhalten werden, dass der vorgegebene spektrale Verlauf auf Basis des ersten und/oder des zweiten ortsaufgelösten optischen Parametersatzes verschoben wird. Dadurch wird beispielsweise der technische Vorteil erreicht, dass sich die Farbwerte des Dentalobjektes auf Basis eines empirisch gemessenen Verlaufs bestimmen und anpassen lassen.

In einer weiteren technisch vorteilhaften Ausführungsform des Verfahrens ist der vorgegebene spektrale Verlauf ein Verlauf eines Reflexionsgrads, eines Absorptionskoeffizienten und/oder eines Streukoeffizienten in Abhängigkeit der Lichtwellenlänge. Dadurch wird beispielsweise der technische Vorteil erreicht, dass besonders geeignete optische Parameter zur Farbbestimmung herangezogen werden.

In einer weiteren technisch vorteilhaften Ausführungsform des Verfahrens ordnet der erste und/oder zweite ortsaufgelöste optische Parametersatz den Orten der Oberfläche des Dentalobjekts jeweils einen oder mehrere optische Parameter zu. Die optischen Parameter an den Orten der Oberfläche schließen auch solche Parameter ein, für die das Licht ein Stück weit in das Dentalobjekt eindringt. Dadurch wird beispielsweise der technische Vorteil erreicht, dass die optischen Parameter für jeden Ort der Oberfläche bei der ersten und der zweiten Lichtwellenlänge bekannt sind.

In einer weiteren technisch vorteilhaften Ausführungsform des Verfahrens wird das erste Streifenlichtmuster mit einer ersten Lichtquelle erzeugt und das zweite Streifenlichtmuster mit einer zweiten Lichtquelle erzeugt. Die Lichtquellen können durch Leuchtdioden oder Laserdioden gebildet sein. Je schmalbandiger die Lichtquelle ist, desto genauer lassen sich die optischen Parameter bei der jeweiligen Lichtwellenlänge bestimmen. Dadurch wird beispielsweise der technische Vorteil erreicht, dass eine genaue ortsaufgelöste Bestimmung der optischen Parameter erreicht wird.

In einer weiteren technisch vorteilhaften Ausführungsform des Verfahrens wird das Verfahren durch einen Intraoralscanner ausgeführt. Dadurch wird beispielsweise der technische Vorteil erreicht, dass sich ortsaufgelöste Farbwerte eines Zahns mit hoher Genauigkeit ermitteln lassen.

Gemäß einem zweiten Aspekt wird die technische Aufgabe durch ein Farbbestimmungssystem zur ortsaufgelösten Farbbestimmung gelöst, mit einer ersten Projektionseinrichtung zum Projizieren eines ersten Streifenlichtmusters mit einer ersten Lichtwellenlänge auf ein Dentalobjekt; einer ersten Erfassungseinrichtung zum Erfassen eines ersten ortsaufgelösten optischen Parametersatzes auf Basis des reflektierten oder remittierten ersten Streifenlichtmusters; einer zweiten Projektionseinrichtung zum Projizieren eines zweiten Streifenlichtmusters einer zweiten Lichtwellenlänge auf das Dentalobjekt; einer zweiten Erfassungseinrichtung zum Erfassen eines zweiten ortsaufgelösten optischen Parametersatzes auf Basis des reflektierten oder remittierten zweiten Streifenlichtmusters; und einer Berechnungseinrichtung zum Berechnen eines dritten ortsaufgelösten optischen Parametersatzes bei einer dritten Lichtwellenlänge auf Basis des ersten und des zweiten ortsaufgelösten optischen Parametersatzes. Die erste und die zweite Projektionseinrichtung können innerhalb eines einzigen Projektionssystems realisiert sein. Die erste und die zweite Erfassungseinrichtung können innerhalb eines einzigen Erfassungssystems realisiert sein. Durch das Farbbestimmungssystem werden die gleichen technischen Vorteile wie durch das Verfahren nach dem ersten Aspekt erreicht.

In einer technisch vorteilhaften Ausführungsform des Farbbestimmungssystems ist das Farbbestimmungssystem ausgebildet, den dritten ortsaufgelösten optischen Parametersatz aus dem ersten und dem zweiten ortsaufgelösten optischen Parametersatz zu extrapolieren und/oder den dritten ortsaufgelösten optischen Parametersatz zwischen dem ersten und dem zweiten ortsaufgelösten optischen Parametersatz zu interpolieren. Dadurch wird beispielsweise ebenfalls der technische Vorteil erreicht, dass sich der dritte Parametersatz durch den linearen Zusammenhang auf einfache und schnelle Weise ermitteln lässt.

In einer weiteren technisch vorteilhaften Ausführungsform des Farbbestimmungssystems ist das Farbbestimmungssystem ausgebildet, einen vorgegebenen spektralen Verlauf an den ersten und den zweiten ortsaufgelösten optischen Parametersatz anzupassen, um den dritten ortsaufgelösten optischen Parametersatz zu erhalten. Dadurch wird beispielsweise ebenfalls der technische Vorteil erreicht, dass sich die Farbwerte des Dentalobjektes auf Basis eines empirisch gemessenen Verlaufs bestimmen und anpassen lassen.

In einer weiteren technisch vorteilhaften Ausführungsform des Farbbestimmungssystems umfasst das Farbbestimmungssystem eine erste Lichtquelle zum Erzeugen des ersten Streifenlichtmusters und eine zweite Lichtquelle zum Erzeugen des zweiten Streifenlichtmusters. Dadurch wird beispielsweise ebenfalls der technische Vorteil erreicht, dass eine genaue ortsaufgelöste Farbbestimmung erreicht wird.

Gemäß einem dritten Aspekt wird die technische Aufgabe durch einen Intraoralscanner mit einem Farbbestimmungssystem nach dem zweiten Aspekt gelöst. Dadurch wird der technische Vorteil erreicht, dass sich Farbwerte innerhalb eines Mundraums, wie beispielsweise von Zähnen, ortsaufgelöst und genau bestimmen lassen.

Ausführungsbeispiele der Erfindung sind in den Zeichnungen dargestellt und werden im Folgenden näher beschrieben.

Es zeigen:

Fig. 1　eine schematische Ansicht eines Farbbestimmungssystems zur ortsaufgelösten Farbbestimmung eines Dentalobjekts;

Fig. 2　ein Blockdiagramm eines Verfahrens zur ortsaufgelösten Farbbestimmung; und

Fig. 3　ein Prinzip eines Korrigierens eines allgemeinen Verlaufs des Absorptionskoeffizienten auf Basis von zwei gemessenen Werten.

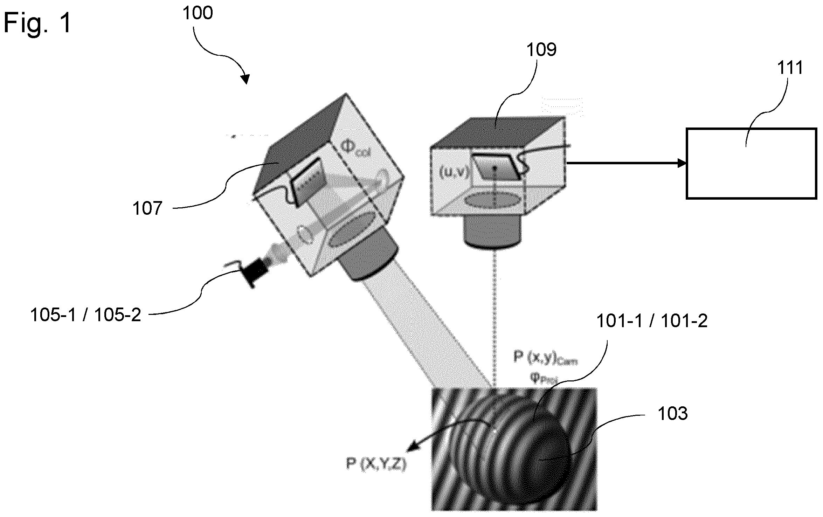

Fig. 1 zeigt eine schematische Ansicht eines Farbbestimmungssystems 100 zur ortsaufgelösten Farbbestimmung eines Dentalobjekts 103. Das Farbbestimmungssystem 100 umfasst eine Projektionseinrichtung 107 als Streifenlichtprojektor, die Streifenlichtmuster 101-1 und 101-2 mit unterschiedlicher Ortsfrequenz und unterschiedlicher Lichtwellenlänge auf das Dentalobjekt 103 projizieren kann. Diese Projektionseinrichtung 107 umfasst beispielsweise ein Mikro-LCD-Display ein digitales Spiegelgerät (Digital Mirror Device - DMD) und eine geeignete Optik.

Beispielsweise projiziert die Projektionseinrichtung 107 Streifenlichtmuster 101-1 mit unterschiedlichen Ortsfrequenzen, d.h. Abständen der Streifen, bei einer ersten Lichtwellenlänge auf das Dentalobjekt 103. Anschließend projiziert die Projektionseinrichtung 107 Streifenlichtmuster 101-1 mit unterschiedlichen Ortsfrequenzen bei einer zweiten Lichtwellenlänge auf das Dentalobjekt 103. Die erste und die zweite Lichtwellenlänge sind hierbei unterschiedlich.

Zur Projektion der Streifenlichtmuster 101-1 und 101-2 kann das Farbbestimmungssystem 100 beispielsweise zwei Lichtquellen 105-1 und 105-2 mit den jeweiligen Lichtwellenlängen umfassen. Die Lichtquellen 105-1 und 105-2 können beispielsweise durch Leuchtdioden oder Laserdioden gebildet sein, die geeignet sind, Licht mit der gewünschten Lichtwellenlänge auszusenden. Im Allgemeinen lassen sich auch weitere Streifenlichtmuster mit weiteren Lichtwellenlängen verwenden.

Aus diesen Streifenlichtmustern 101-1 und 101-2 lässt sich zum einen die räumliche Form des Dentalobjekts 103 ermitteln. Zu diesem Zweck werden die von dem Dentalobjekt 103 reflektierten oder remittierten Streifenlichtmuster 101-1 und 101-2 durch eine Erfassungseinrichtung 109 erfasst, die durch eine elektronische Kamera mit einem CCD-Chip gebildet sein kann. Die erfassten Aufnahmen der reflektierten oder remittierten Streifenlichtmuster 101-1 und 101-2 werden durch einen geeigneten Algorithmus ausgewertet, der aus dem Verlauf des reflektierten oder remittierten Streifenlichtmusters die räumliche Form des Dentalobjektes 103 berechnen kann. Hierzu wird eine Triangulation mit einem Winkel zwischen Einfall und Remission durchgeführt.

Dieser Algorithmus wird auf einem digitalen Prozessor 111 ausgeführt. Die gewonnenen Daten über die räumliche Form des Dentalobjekts 103 können in einem digitalen Speicher abgelegt werden.

Zum anderen lassen sich aber auch optische Parameter an jedem Ort der Oberfläche des Dentalobjektes 103 bei derjenigen Lichtwellenlänge ermitteln, die für das Licht des jeweiligen Streifenlichtmusters 101-1 und 101-2 verwendet wird. Daraus können ortsaufgelöste optische Parametersätze erhalten werden, die die optischen Eigenschaften für jeden Ort der Oberfläche des Dentalobjekts 103 bei dieser Lichtwellenlänge angeben.

Durch eine quantitative und ortsaufgelöste Messung der remittierten und reflektierten Intensitätsamplitude und Phasenverschiebung können modellgestützt durch Lösung der Strahlungstransporttheorie die für die Lichtausbreitung relevanten Größen bestimmt werden, wie beispielsweise der Reflexionsgrad, der effektive Streukoeffizient und der Absorptionskoeffizient.

Generell beruht die Idee der Farbmessung mit Streifenlicht auf der Tatsache, dass die optischen Eigenschaften eines Materials ortsaufgelöst berechnet werden können, d.h. über die Fläche des beleuchteten Materials. In diesem Fall wird ein Streifenmuster auf das Material projiziert und das remittierte Licht mit einer Kamera gemessen. Anschließend können die optischen Parameter des Materials mit Modellrechnungen berechnet werden, die beispielsweise auf dem Lösen der Strahlungstransportgleichung beruhen.

Beispielsweise ist der optische Parameter an einem Ort des Dentalobjektes 103 höher als an einem anderen Ort des Dentalobjektes 103. Dieser optische Parameter lässt sich ortsaufgelöst beispielsweise aus der Intensität der reflektierten oder remittierten Streifen der Streifenlichtmuster 101-1 und 101-2 ermitteln. Der optische Parametersatz umfasst dann beispielsweise einen ortsaufgelösten Reflexionsgrad, einen Absorptions- und/oder Streukoeffizienten.

Sind ein erster ortsaufgelöster optischer Parametersatz bei der ersten Lichtwellenlänge und ein zweiter ortsaufgelöster optischer Parametersatz bei der zweiten Lichtwellenlänge bekannt, so lässt sich aus diesen ein dritter ortsaufgelöster Parametersatz für eine dritte Lichtwellenlänge berechnen. In diesem Fall kann auf eine Messung mittels einer weiteren Lichtquelle 105 verzichtet werden. Dadurch verringert sich die Anzahl der Lichtquellen 105 innerhalb des Farbbestimmungssystems 100.

Um die Anzahl der Lichtquellen 105 zu begrenzen, können bei Kenntnis des zu vermessenden Materials, d.h. beispielsweise einem natürlichen Zahn, die verwendeten Lichtwellenlängen zudem geschickt auswählt werden. Beispielsweise kann mit zwei Streifenlichtmustern 101-1 und 101-2 mit Lichtwellenlängen von 450 nm und 600 nm zunächst eine erste Steigung zwischen den jeweiligen Punkten der ortsaufgelösten Parametersätze bei 450 nm und 600 nm bestimmt werden. Dann können mit zwei weiteren Streifenlichtmustern mit Lichtwellenlängen bei 520 nm und 650 nm die charakteristischen Minima in der Reflexionskurve vermessen werden. In diesem Fall werden vier Lichtquellen 105 für die Projektion der jeweiligen Streifenlichtmuster 101 verwendet.

Eine genaue Auswahl der Lichtquellen 105 mit geeigneten Lichtwellenlängen kann auf unterschiedliche Weise erfolgen. Beispielsweise kann eine Untersuchung der optischen Eigenschaften zahlreicher Restaurationsmaterialien oder Zahnmaterialien erfolgen, um charakteristische Lichtwellenlängen zu finden, an denen Unterschiede im Wellenlängenspektrum zu erwarten sind. Die Streifenlichtmuster 101-1 und 101-2 werden anschließend mit Licht dieser gefundenen Lichtwellenlänge projiziert. Zu jedem untersuchten Restaurationsmaterial oder Zahnmaterial lassen sich die charakteristischen Lichtwellenlängen in einer Datenbank ablegen. Die Auswahl der zu verwendenden Lichtquellen 105 kann dann auf Basis der Datenbank und sinnvoller Stützpunktwellenlängen durchgeführt werden.

Zudem können optische Daten ausgewertet werden, die als Reflexionsspektrum in vivo bei möglichst vielen Menschen gemessen worden sind, um charakteristische Lichtwellenlängen zu finden. Je nach Region, in der die Menschen leben, weisen die Reflexionsspektren unterschiedliche charakteristische Lichtwellenlängen auf, d.h. unterschiedliche durchschnittliche Zahnfarben. In diesem Fall kann die Lichtwellenlänge der Lichtquellen 105 auf die jeweilige Region angepasst werden.

Damit kann eine ortsaufgelöste und präzise Farbmessung erreicht werden, da die spektrale Auflösung ausreicht, d.h. die Anzahl der Spektralpunkte für die zur Streifenlichtprojektion verwendeten Farben, um aus den ortsaufgelösten optischen Parametersätzen ein möglichst kontinuierliches Spektrum für jeden Ort auf der Oberfläche des Dentalobjektes 103 zu berechnen. Dieses kontinuierliche Spektrum für jeden Ort der Oberfläche kann dann in L*a*b*-Werte umgerechnet werden. Der L*a*b*-Farbraum (auch: CIELAB, CIEL*a*b*, Lab-Farben) beschreibt alle wahrnehmbaren Farben. Dieser verwendet einen dreidimensionalen Farbenraum, bei dem der Helligkeitswert L* senkrecht auf der Farbebene (a*, b*) steht.

Zunächst werden aus dem Reflexionsspektrum X-, Y-, und Z-Werte berechnet, aus denen dann und dann L*a*b* errechnet werden:

$$X = \frac{1}{N} \int_\lambda x(\lambda)S(\lambda)I(\lambda)d\lambda$$

$$Y = \frac{1}{N} \int_\lambda y(\lambda)S(\lambda)I(\lambda)d\lambda$$

$$Z = \frac{1}{N} \int_\lambda z(\lambda)S(\lambda)I(\lambda)d\lambda$$

$$N = \int_\lambda y(\lambda)I(\lambda)d\lambda$$

Werden Streifenmuster 101-1 und 101-2 mit je einer speziellen optischen Lichtwellenlänge verwendet, erhält einen ortsaufgelösten optischen Parametersatz für genau diese Lichtwellenlänge. Ausreichend viele Berechnungen mit anderen Lichtwellenlängen können dann weitere ortsaufgelöste optische Parametersätze über das gesamte sichtbare Spektrum ergeben. Somit erhält man für jeden Ort einen spezifischen kontinuierlichen Verlauf der optischen Parameter.

Auf diese Weise werden L*a*b*-Farbwerten erhalten, wenn ein Reflexionsspektrum gemessen worden ist, das noch im direkten Vergleich mit weißer (idealer Streuer) und schwarzer (minimale Reflexion) Referenz korrigiert worden ist. Wenn die Absorptions- und Streukoeffizienten bekannt sind, muss nicht unbedingt erst ein Reflexionsspektrum berechnet werden, um dann daraus L*a*b* Farbwerte zu erzeugen.

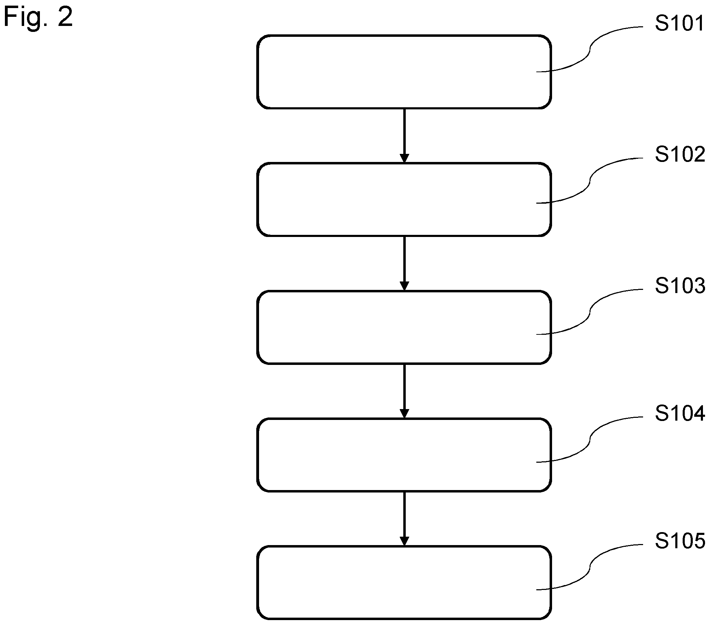

Fig. 2 zeigt ein Blockdiagramm eines Verfahrens zur ortsaufgelösten Farbbestimmung. Im Schritt S101 werden ein oder mehrere erste Streifenlichtmuster 101-1 mit der ersten Lichtwellenlänge auf das Dentalobjekt 103 projiziert. Hierbei können unterschiedliche und variierende Ortsfrequenzen des Streifenlichtmusters 101-1 verwendet werden, solange die Lichtwellenlänge des Streifenlichtmusters 101-1 unverändert bleibt. Aus den ersten Streifenlichtmustern 101-1, die von dem Dentalobjekt 103 reflektiert werden, kann eine räumliche Form des Dentalobjekts 103 bestimmt werden. Hierzu wertet ein Algorithmus den Verlauf der Streifen in dem reflektierten oder remittierten Streifenlichtmuster 101-1 aus.

Allerdings kann die 3D-Geometrie auch bei möglichst kurzen Wellenlängen (blaues Licht) bestimmt werden, um die Eindringtiefe minimal zu halten. Außerdem zum Beispiel nichtperiodische Muster verwendet werden, um eine Streifenzuordnung besser durchzuführen.

In Schritt S102 wird zusätzlich ein erster ortsaufgelöster optischer Parametersatz auf Basis der ersten Streifenlichtmuster 101-1 bestimmt, die von dem Dentalobjekt 103 reflektiert oder remittiert werden. Der erste ortsaufgelöste optische Parametersatz umfasst beispielsweise ortsaufgelöste Werte für einen Reflexionsgrad, einen Absorptions- und/oder Streukoeffizienten bei der ersten Lichtwellenlänge. Durch das Streifenlichtverfahren wird daher nicht nur die räumliche Form des Dentalobjektes 103 bestimmt, sondern auch eine Verteilung eines optischen Parameters auf der Oberfläche des Dentalobjektes 103 bei der ersten Lichtwellenlänge ermittelt. Jedem Ort auf der Oberfläche des Dentalobjekts 103 kann zumindest ein optischer Parameter zugeordnet werden. Die Gesamtheit dieser optischen Parameter für die jeweiligen Orte ist in dem ersten ortsaufgelösten optischen Parametersatz umfasst.

Im Schritt S103 werden zudem ein oder mehrere zweite Streifenlichtmuster 101-2 mit der zweiten Lichtwellenlänge auf das Dentalobjekt 103 projiziert. Hierbei können ebenfalls unterschiedliche und variierende Ortsfrequenzen des Streifenlichtmusters 101-2 verwendet werden, solange die Lichtwellenlänge der Streifenmuster 101-2 unverändert bleibt. In diesem Fall wird die Messung bei einer anderen Lichtwellenlänge als bei der ersten Lichtwellenlänge durchgeführt. Aus den zweiten Streifenlichtmustern 101-2, die von dem Dentalobjekt 103 reflektiert oder remittiert werden, können ebenfalls die räumliche Form des Dentalobjekts 103 bestimmt werden.

In Schritt S104 wird ein zweiter ortsaufgelöster optischer Parametersatz auf Basis der zweiten Streifenlichtmuster 101-2 bestimmt, die von dem Dentalobjekt 103 reflektiert oder remittiert werden. Aus diesen ortsaufgelösten optischen Parametersatz ergibt sich eine weitere Verteilung der optischen Eigenschaften über die Oberfläche des Dentalobjektes 103 bei der zweiten Lichtwellenlänge. Der zweite ortsaufgelöste optische Parametersatz umfasst beispielsweise ortsaufgelöste Werte für einen Reflexionsgrad, einen Absorptions- und/oder Streukoeffizienten bei der zweiten Lichtwellenlänge in entsprechender Weise zu dem ersten ortsaufgelösten optischen Parametersatz. Die Gesamtheit dieser optischen Parameter für die jeweiligen Orte ist in dem zweiten ortsaufgelösten optischen Parametersatz umfasst.

In Schritt S105 wird ein dritter ortsaufgelöster optischer Parametersatzes bei der dritten Lichtwellenlänge auf Basis des ersten und des zweiten ortsaufgelösten optischen Parametersatzes berechnet. Der dritte ortsaufgelöste optische Parametersatz gibt den optischen Parameter an, wie wenn dieser bei der dritten Lichtwellenlänge gemessen worden wäre. Da dieser jedoch berechnet wird, kann auf die Verwendung einer Lichtquelle 105 hierzu verzichtet werden. Das Verfahren zur Berechnung des dritten ortsaufgelösten optischen Parametersatzes wird durch die Berechnungseinrichtung 111 durchgeführt.

Dies kann beispielsweise auf einfache Weise dadurch geschehen, dass für die dritte Lichtwellenlänge zwischen dem ersten ortsaufgelösten optischen Parametersatz und dem zweiten ortsaufgelösten optischen Parametersatz im Wellenlängenbereich inter- oder extrapoliert wird. Durch die Interpolation oder Extrapolation ist es beispielsweise möglich, für jeden Wellenlängenwert die entsprechenden optische Eigenschaften zu berechnen. Die Interpolation oder Extrapolation kann für jeden Ort auf der Oberfläche des Dentalobjektes 103 durchgeführt werden. Dadurch kann zu jedem Ort auf der Oberfläche des Dentalobjektes 103 ein kontinuierliches Spektrum erhalten werden.

Beispielsweise kann aus zwei gemessenen Werten des Absorptionskoeffizienten $\alpha_{x1,y1,z1,\lambda 1}$ am Ort x1, y1 und z1 bei der Lichtwellenlänge $\lambda 1$ und des Absorptionskoeffizienten $\alpha_{x1,y1,z1,\lambda 2}$ am gleichen Ort x1, y1 und z1 bei der Lichtwellenlänge $\lambda 2$ ein allgemeiner Verlauf des Absorptionskoeffizienten $\alpha_{x1,y1,z1}(\lambda)$ in Abhängigkeit der Lichtwellenlänge am Ort x1, y1 und z1 bestimmt werden.

Im einfachsten Fall wird hierbei zwischen den beiden gemessenen Absorptionskoeffizienten $\alpha_{x1,y1,z1,\lambda 1}$ und $\alpha_{x1,y1,z1,\lambda 2}$ interpoliert oder die beiden gemessenen Absorptionskoeffizienten $\alpha_{x1,y1,z1,\lambda 1}$ und $\alpha_{x1,y1,z1,\lambda 2}$ werden extrapoliert. Dadurch wird ein allgemeiner linearer Zusammenhang $\alpha_{x1,y1,z1}(\lambda)$ in Abhängigkeit der Lichtwellenlänge am Ort x1, y1 und z1 hergestellt.

Wird dieses Verfahren für alle Orte x, y, z auf der Oberfläche des Dentalobjektes 103 durchgeführt, so wird aus dem ersten und zweiten ortsaufgelösten optischen Parametersatz ein dritter ortsaufgelöster optischer Parametersatz bei jeder gewünschten dritten Lichtwellenlänge erhalten. Auf eine Lichtquelle 105 bei dieser Lichtwellenlänge kann dann verzichtet werden, so dass sich der Bauraum entsprechend verringert.

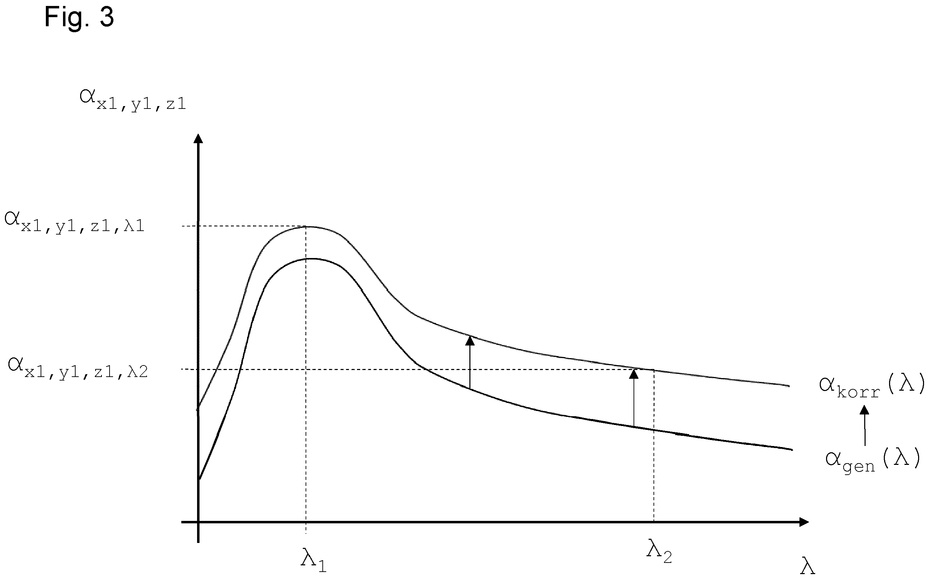

Fig. 3 zeigt das Prinzip eines Korrigierens eines allgemeinen Verlaufs des Absorptionskoeffizienten auf Basis von zwei gemessenen Werten, nämlich dem Absorptionskoeffizient $\alpha_{x1,y1,z1,\lambda 1}$ am Ort x1, y1 und z1 bei der Lichtwellenlänge $\lambda 1$ und des Absorptionskoeffizienten $\alpha_{x1,y1,z1,\lambda 2}$ am gleichen Ort x1, y1 und z1 bei der Lichtwellenlänge $\lambda 2$. Durch dieses Verfahren kann ein dritter ortsaufgelöster optischer Parametersatzes bei einer beliebigen dritten Lichtwellenlänge $\lambda 3$ erhalten werden, wie wenn dieses für die entsprechenden Orte der Oberfläche des Dentalobjektes 103 ausgeführt wird.

Hierzu kann ein allgemeiner generischer Verlauf des Absorptionskoeffizienten $\alpha_{gen}(\lambda)$ für beispielsweise einen Zahn vorgegeben sein, der an die beiden gemessenen Absorptionskoeffizienten $\alpha_{x1,y1,z1,\lambda 1}$ und $\alpha_{x1,y1,z1,\lambda 2}$ angepasst wird, beispielsweise durch Subtraktion einer Differenz zu den gemessenen Absorptionskoeffizienten $\alpha_{x1,y1,z1,\lambda 1}$ und $\alpha_{x1,y1,z1,\lambda 2}$ oder durch ein Fitverfahren. Dem Fitverfahren kann ein zu erwartender funktionaler Zusammenhang zwischen dem optischen Parameter und der Lichtwellenlänge zugrunde gelegt werden. Durch das Fitverfahren werden Fitparameter des funktionalen Zusammenhangs so verändert, dass die gemessenen Absorptionskoeffizienten $\alpha_{x1,y1,z1,\lambda 1}$ und $\alpha_{x1,y1,z1,\lambda 2}$ möglichst nahe an der Fitfunktion liegen.

Der vorgegebene Verlauf kann beispielsweise auch auf einem zuvor gemessenen empirischen Spektrum eines ähnlichen Dentalobjekts 103 oder auf einem prinzipiellen funktionalen Verlauf beruhen, der für diese Klasse von Dentalobjekten 103 in ähnlicher Form an allen Orten der Oberfläche des Dentalobjekts 103 beobachtet wird.

Aus dem allgemeinen Verlauf des Absorptionskoeffizienten $\alpha_{gen}(\lambda)$ kann jeder Absorptionskoeffizient $\alpha_{x1,y1,z1,\lambda 3}$ bei einer beliebigen anderen Lichtwellenlänge $\lambda 3$ berechnet werden. Zu diesem Zweck wird beispielsweise der allgemeine Verlauf des Absorptionskoeffizienten $\alpha_{gen}(\lambda)$ an die beiden zuvor gemessenen Absorptionskoeffizienten $\alpha_{x1,y1,z1,\lambda 1}$ und $\alpha_{x1,y1,z1,\lambda 2}$ angepasst, so dass ein korrigierter Verlauf $\alpha_{korr}(\lambda)$ erhalten wird. Beispielsweise kann der allgemeine Verlauf des Absorptionskoeffizienten $\alpha_{gen}(\lambda)$ auf Basis der beiden gemessenen Absorptionskoeffizienten $\alpha_{x1,y1,z1,\lambda 1}$ und $\alpha_{x1,y1,z1,\lambda 2}$ verschoben, geschert, gestaucht, gedreht oder auf andere Weise korrigiert werden.

Aus dem ersten und dem zweiten ortsaufgelösten optischen Parametersatz können daher eine beliebige Anzahl dritter ortsaufgelöster optischer Parametersätze bei jeder beliebigen Lichtwellenlänge $\lambda 3$ berechnet werden. Auf diese Weise erhält man zu jedem Punkt x, y, und z auf der Oberfläche des Zahnes einen vollständigen quasikontinuierlichen spektralen Verlauf des Absorptionskoeffizienten α und es lässt sich für jeden Punkt der Oberfläche eine vollständige spektrale Information erhalten. Dieses Verfahren funktioniert nicht nur mit dem Absorptionskoeffizienten α, sondern auch mit anderen optischen Parametern. Das Verfahren ermöglich prinzipiell eine präzise Farbmessung für jedes Objekt, bei dem ein ungefährer Verlauf der optischen Parameter im Voraus bekannt ist.

Bei der Verwendung verschiedener Ortsfrequenzen des Streifenlichtmusters können zudem optische Informationen aus unterschiedlichen Tiefen des Dentalobjektes 103 erhalten werden. Mit diesen Informationen kann eine Bestimmung des Reflexionsgrads, des Absorptions- und/oder des Streukoeffizienten weiter verbessert werden.

Mit diesem Verfahren wird nicht nur eine Punktmessung der Farbeigenschaften durchgeführt, sondern es werden flächige Farbmessungen durchgeführt, beispielsweise für zahlreiche Punkte in einem Bereich von 10 x 10 mm. Dies führt dazu, dass zu jedem dieser Punkte dessen optische Parameter bei jeder Lichtwellenlänge berechnet werden können. Dadurch wird zu jedem Punkt der Oberfläche ein vollständiges und kontinuierliches Farbspektrum erhalten, für das der optische Parameter in beliebig kleiner spektraler Auflösung erhalten werden kann. Auf eine Verwendung unvollständiger Reflexionsspektren kann verzichtet werden.

Im Falle eines Zahns als Dentalobjekt 103 wird die Transluzenz eines Schmelzes automatisch mitbestimmt, da diese nur eine Manifestation der intrinsischen optischen Eigenschaften Absorption und Streuung darstellt. In den tiefenaufgelösten Messungen bei unterschiedlichen Ortsfrequenzen verbergen sich Absorptions- und Streueigenschaften des Zahnschmelzes. Daher ist die Transparenz, auch Transluzenz genannt, intrinsisch mit bekannt. Neben den L*a*b*-Werten kann die Transluzenz verwendet werden, um einen natürlich aussehenden Zahnersatz zu erzielen.

Ein weiterer technischer Vorteil ist, dass mit dem Verfahren winkelabhängig gemessen werden kann. Der Winkel verändert die Farbmessung an einem menschlichen Zahn und der Gingiva aufgrund der unterschiedlichen Umgebung. Durch die Kombination mit den dreidimensionalen Daten für das Dentalobjekt 103 ist es möglich, den Winkel für jedes Paar von Raum- und Farbdaten zu bestimmen. Daraus kann ein genaues digitales räumliches Abbild eines Zahnes in Verbindung mit der Farbmessung erhalten werden. Durch eine Vermessung der dreidimensionalen Topografie des Dentalobjekts 103 kann die Berechnung zusätzlich verbessert werden.

Mit dem Verfahren zur ortsaufgelösten Farbbestimmung können nicht nur intraoral die optischen Parameter von Zähnen bestimmt werden, sondern auch extraoral die optischen Parameter von dentalen Restaurationen, wie beispielsweise Veneers, Kronen, Brücken, Inlays oder Onlays. Durch einen Abgleich mit Werten aus einer Datenbank kann außerdem eine Restaurations- und Materialerkennung durchgeführt werden.

Das Verfahren zur ortsaufgelösten Farbbestimmung kann in einem auswechselbaren Aufsatz eines Intraoralscanners durchgeführt werden. Auch eine Licht- und Streifenmustererzeugung kann im auswechselbaren Aufsatz des Intraoralscanners stattfinden. Eine Streifenmustererzeugung kann in dem Aufsatz des Intraoralscanners mittels Mikro-LCD-Display durchgeführt werden. Die Aufnahme der Bilder der reflektierten oder remittierten Streifenlichtmuster kann durch einen Kamerachip im Hauptgehäuse des Intraoralscanners erfolgen.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

BEZUGSZEICHENLISTE

| | |
|---|---|
| 100 | Farbbestimmungssystems |
| 101 | Streifenlichtmuster |
| 103 | Dentalobjekt |
| 105 | Lichtquelle |
| 107 | Projektionseinrichtung |
| 109 | Erfassungseinrichtung |
| 111 | Berechnungseinrichtung |

**Patentansprüche**

1. Verfahren zur ortsaufgelösten Farbbestimmung, mit den Schritten:

- Projizieren (S101) eines ersten Streifenlichtmusters (101-1) mit einer ersten Lichtwellenlänge auf ein Dentalobjekt (103);
- Erfassen (S102) eines ersten ortsaufgelösten optischen Parametersatzes auf Basis des reflektierten oder remittierten ersten Streifenlichtmusters (101-1);
- Projizieren (S103) eines zweiten Streifenlichtmusters (101-2) mit einer zweiten Lichtwellenlänge auf das Dentalobjekt (103);
- Erfassen (S104) eines zweiten ortsaufgelösten optischen Parametersatzes auf Basis des reflektierten oder remittierten zweiten Streifenlichtmusters (101-2); und

- Berechnen (S105) eines dritten ortsaufgelösten optischen Parametersatzes bei einer dritten Lichtwellenlänge auf Basis des ersten und des zweiten ortsaufgelösten optischen Parametersatzes.

**2.** Verfahren nach Anspruch 1, wobei der dritte ortsaufgelöste optische Parametersatz aus dem ersten und dem zweiten ortsaufgelösten optischen Parametersatz extrapoliert wird.

**3.** Verfahren nach einem der vorangehenden Ansprüche, wobei der dritte ortsaufgelöste optische Parametersatz zwischen dem ersten und dem zweiten ortsaufgelösten optischen Parametersatz interpoliert wird.

**4.** Verfahren nach einem der vorangehenden Ansprüche, wobei der dritte Parametersatz durch ein Fitverfahren auf Basis des ersten und des zweiten ortsaufgelösten optischen Parametersatzes bestimmt wird.

**5.** Verfahren nach einem der vorangehenden Ansprüche, wobei ein vorgegebener spektraler Verlauf an den ersten und den zweiten ortsaufgelösten optischen Parametersatz angepasst wird, um den dritten ortsaufgelösten optischen Parametersatz zu erhalten.

**6.** Verfahren nach einem der vorangehenden Ansprüche, wobei der vorgegebene spektrale Verlauf ein Verlauf eines Reflexionsgrads, eines Absorptionskoeffizienten und/oder eines Streukoeffizienten in Abhängigkeit der Lichtwellenlänge ist.

**7.** Verfahren nach einem der vorangehenden Ansprüche, wobei der erste und/oder zweite ortsaufgelöste optische Parametersatz den Orten der Oberfläche des Dentalobjekts (103) jeweils einen oder mehrere optische Parameter zuordnet.

**8.** Verfahren nach einem der vorangehenden Ansprüche, wobei das erste Streifenlichtmuster (101-1) mit einer ersten Lichtquelle (105-1) erzeugt wird und das zweite Streifenlichtmuster (101-2) mit einer zweiten Lichtquelle (105-2) erzeugt wird.

**9.** Verfahren nach einem der vorangehenden Ansprüche, wobei das Verfahren durch einen Intraoralscanner ausgeführt wird.

**10.** Farbestimmungssystem (100) zur ortsaufgelösten Farbbestimmung, mit:

- einer ersten Projektionseinrichtung (107) zum Projizieren eines ersten Streifenlichtmusters (101-1) mit einer ersten Lichtwellenlänge auf ein Dentalobjekt (103);
- einer ersten Erfassungseinrichtung (109) zum Erfassen (S102) eines ersten ortsaufgelösten optischen Parametersatzes auf Basis des reflektierten oder remittierten ersten Streifenlichtmusters (101-1);
- einer zweiten Projektionseinrichtung (107) zum Projizieren (S103) eines zweiten Streifenlichtmusters (101-2) einer zweiten Lichtwellenlänge auf das Dentalobjekt (103);
- einer zweiten Erfassungseinrichtung (109) zum Erfassen (S104) eines zweiten ortsaufgelösten optischen Parametersatzes auf Basis des reflektierten oder remittierten zweiten Streifenlichtmusters (101-2); und
- einer Berechnungseinrichtung (111) zum Berechnen (S105) eines dritten ortsaufgelösten optischen Parametersatzes bei einer dritten Lichtwellenlänge auf Basis des ersten und des zweiten ortsaufgelösten optischen Parametersatzes.

**12.** Farbbestimmungssystem (100) nach Anspruch 11, wobei das Farbbestimmungssystem (100) ausgebildet ist, den dritten ortsaufgelösten optischen Parametersatz aus dem ersten und dem zweiten ortsaufgelösten optischen Parametersatz zu extrapolieren und/oder den dritten ortsaufgelösten optischen Parametersatz zwischen dem ersten und dem zweiten ortsaufgelösten optischen Parametersatz zu interpolieren.

**13.** Farbestimmungssystem (100) nach einem der Ansprüche 10 bis 12, wobei das Farbbestimmungssystem (100) ausgebildet ist, einen vorgegebenen spektralen Verlauf an den ersten und den zweiten ortsaufgelösten optischen Parametersatz anzupassen, um den dritten ortsaufgelösten optischen Parametersatz zu erhalten.

**14.** Farbestimmungssystem (100) nach einem der Ansprüche 10 bis 13, wobei das Farbbestimmungssystem (100) eine erste Lichtquelle (105-1) zum Erzeugen des ersten Streifenlichtmusters (101-1) und eine zweite Lichtquelle (105-1) zum Erzeugen des zweiten Streifenlichtmusters (101-1) umfasst.

**15.** Intraoralscanner mit einem Farbestimmungssystem (100) nach einem der Ansprüche 10 bis 14.

Fig. 1

EP 4 311 519 A1

Fig. 2

S101

S102

S103

S104

S105

Fig. 3

Europäisches Patentamt
European Patent Office
Office européen des brevets

**EUROPÄISCHER RECHERCHENBERICHT**

**Nummer der Anmeldung**

**EP 22 18 7237**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2012/231420 A1 (WONG VICTOR C [US] ET AL) 13. September 2012 (2012-09-13) * Abbildung 3 * ----- | 1-14 | INV.<br>A61C13/08<br>A61B5/103<br>G01J3/28<br>G01J3/50 |
| X | US 2020/132547 A1 (YU GEORGE YANG [US]) 30. April 2020 (2020-04-30) * Anspruch 1 * ----- | 1,10 | |

**RECHERCHIERTE SACHGEBIETE (IPC)**

A61C
A61B
G01J

Der vorliegende Recherchenbericht wurde für alle Patentansprüche erstellt

| Recherchenort | Abschlußdatum der Recherche | Prüfer |
|---|---|---|
| München | 11. Januar 2023 | Rödig, Christoph |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

............................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**
EP 22 18 7237

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-01-2023

| Im Recherchenbericht angeführtes Patentdokument | Datum der Veröffentlichung | Mitglied(er) der Patentfamilie | Datum der Veröffentlichung |
|---|---|---|---|
| US 2012231420 A1 | 13-09-2012 | CN 102327156 A | 25-01-2012 |
| | | EP 2407762 A1 | 18-01-2012 |
| | | JP 2012020130 A | 02-02-2012 |
| | | KR 20120006941 A | 19-01-2012 |
| | | US 2012014572 A1 | 19-01-2012 |
| | | US 2012231420 A1 | 13-09-2012 |
| US 2020132547 A1 | 30-04-2020 | CN 113454429 A | 28-09-2021 |
| | | EP 3874244 A1 | 08-09-2021 |
| | | US 2020132547 A1 | 30-04-2020 |
| | | WO 2020092537 A1 | 07-05-2020 |

EPO FORM P0461

Für nähere Einzelheiten zu diesem Anhang : siehe Amtsblatt des Europäischen Patentamts, Nr.12/82